# EUROPEAN PATENT SPECIFICATION

(11) **EP 0 541 178 B1**
(45) Date of publication and mention of the grant of the patent: **20.03.1996**
(21) Application number: 92203391.5
(22) Date of filing: 04.11.1992
(51) Int. Cl.: C07C 209/60

(54) **Secondary amines and a process for their preparation**
Sekundäre Amine und ein Verfahren zu deren Herstellung
Amines secondaires et un procédé pour leur préparation

(30) Priority: 07.11.1991 EP 91310315
(43) Date of publication of application: 12.05.1993
(73) Proprietor: SHELL INTERNATIONALE RESEARCH MAATSCHAPPIJ B.V., NL-2596 HR Den Haag (NL)
(72) Inventor: Van Zon, Arie, NL-1031 CM Amsterdam (NL); Van Asselen, Otto Leonardus Johannes, NL-1031 CM Amsterdam (NL); Drent, Eit, NL-1031 CM Amsterdam (NL)

(56) References cited:
- EP-A- 0 457 386
- FR-A- 2 340 959
- GB-A- 2 136 438

## Description

The present invention relates to a process for preparing secondary amines, secondary amines obtainable by said process, and lubricating oil compositions, fuel compositions and additive concentrates containing said amines.

It is known to prepare secondary amines by reacting ammonia, or a primary amine with carbon monoxide, hydrogen and an olefinic compound. For example, US-A-4,503,217 discloses an aminomethylation process which comprises reacting a polymeric polyolefin, ammonia, primary or secondary amine and synthesis gas in the presence of a ruthenium-containing catalyst system and a single or two-phase solvent medium, heating the mixture to a temperature of at least 100°C and a pressure of at least 100 psi (689 kPa) until there is substantial formation of the desired polyamine and separating said polymeric polyamine product by a phase separation technique. The resulting polymeric products are useful as surfactants, surfactant precursors and wet strength agents for paper products.

US-A-4,832,702 discloses a fuel or lubricant composition which contains one or more polybutyl- or polyisobutylamines of the general formula I 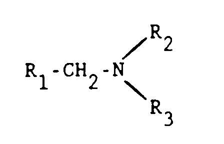
where R₁ is a polybutyl- or polyisobutyl radical derived from isobutene and up to 20% by weight of n-butene and R₂ and R₃ are identical or different and are each hydrogen, an aliphatic or aromatic hydrocarbon, a primary or secondary, aromatic or aliphatic aminoalkylene radical or polyaminoalkylene radical, a polyoxyalkylene radical or a hetaryl or heterocyclyl radical, or, together with the nitrogen atom to which they are bonded, form a ring in which further hetero atoms may be present. The compounds of general formula I are prepared via a two-step process:
i) hydroformylating an appropriate polybutene or polyisobutene with a rhodium or cobalt catalyst in the presence of CO and H₂ at from 80 to 200°C and CO/H₂ pressures of up to 600 bar (60,000 kPa), and
ii) subjecting the oxo product to a Mannich reaction or amination under hydrogenating conditions. The amination reaction is advantageously carried out at 80 to 200°C and under pressures up to 600 bar (60,000 kPa), preferably 80 to 300 bar (8,000 to 30,000 kPa).

US-A-4,526,936 discloses a method for forming polymeric products having a hydrocarbon backbone and a high degree of alkylene alkylamine pendant groups by contacting, in a liquid media, a polymer having unsaturated groups therein, a primary or secondary amine, carbon monoxide and hydrogen gas in the presence of a Group VIII metal containing compound. The formed product is useful for a variety of applications, for example, as a surfactant, a flocculating agent, softener and as a component in coating compositions.

EP-A-457,386 discloses a process for the preparation of an organic compound having at least one secondary amine group, which process comprises reacting ammonia or a primary amine with carbon monoxide, a hydrogen source and an olefinically unsaturated compound in the presence of a catalyst system comprising a source of cationic ruthenium, an aromatic N-heterocyclic ligand and a source of an anion other than a halide.

It has now surprisingly been found that secondary amines can be derived from certain star-shaped polymers with a high conversion and selectivity using certain rhodium catalysts.

In accordance with the present invention there is provided a process for the preparation of a secondary amine which comprises reacting carbon monoxide, hydrogen, a selectively hydrogenated star-shaped polymer comprising a polyvinylaromatic nucleus and at least 4 polymeric arms linked to said nucleus wherein said polymeric arms are selected from the group consisting of:
(i) hydrogenated homopolymers and hydrogenated copolymers of conjugated dienes;
(ii) hydrogenated copolymers of conjugated dienes and monoalkenyl arenes; and
(iii) mixtures thereof;
   and a primary amine derivative having a terminal -NH₂ group and a terminal -NR¹R² group, wherein each of R¹ and R² independently represents a hydrogen atom or an alkyl group; in the presence of a suitable solvent and a catalyst system comprising a source of cationic rhodium; optionally a source of cationic ruthenium; an aromatic N-heterocyclic ligand and a source of an anion other than a halide.

In this specification, unless otherwise stated, an alkyl group may be linear or branched and preferably contains up to 20, more preferably up to 10, and especially up to 6, carbon atoms.

The selectively hydrogenated star-shaped polymer used in the process of the present invention has a number average molecular weight (Mₙ) as determined by gel permeation chromatography on a polystyrene scale preferably in the range of from 25,000 to 1,250,000 and more preferably from 100,000 to 500,000.

Examples of suitable conjugated dienes include butadiene (1,3-butadiene); isoprene; 1,3-pentadiene (piperylene); 2,3-dimethyl-1,3-butadiene; 3-butyl-1,3-octadiene; 1-phenyl-1,3-butadiene; 1,3-hexadiene; and 4-ethyl-1,3-hexadiene. Preferred conjugated dienes are butadiene and isoprene.

Examples of suitable monoalkenyl arene compounds include monovinyl aromatic compounds such as styrene, monovinylnaphthalene as well as the alkylated derivatives thereof such as o-, m- and p-methylstyrene, alphamethylstyrene and tertiary-butylstyrene. Styrene is the preferred monoalkenyl arene compound.

The number of polymeric arms may vary considerably but is typically in the range from 4 to 25, preferably from 7 to 15.

The star-shaped polymer is generally produced by the process comprising the following reaction steps:
(i) polymerising one or more conjugated dienes and, optionally, one or more monoalkenyl arene compounds, in solution in the presence of an ionic initiator to form a living polymer;
(ii) reacting the living polymer with a polyvinyl aromatic compound, preferably divinyl benzene, to form a star-shaped polymer; and
(iii) hydrogenating the star-shaped polymer to form a hydrogenated star-shaped polymer.

This preparation is described in detail in GB-A-1,575,507.

The primary amine derivative may consist of carbon, hydrogen and nitrogen atoms or it may additionally contain oxygen and/or sulphur atoms. For example, the primary amine derivative may be a compound of the general formula: 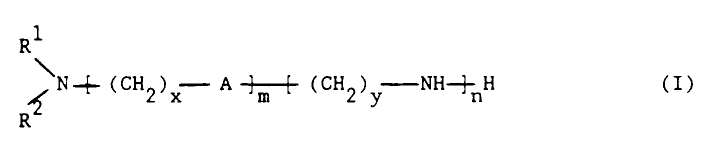
wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and R¹ and R² are as defined above.

In the primary amine derivative of formula I above, it is preferred that each of R¹ and R² independently represents a hydrogen atom or a C₁-C₆ alkyl, especially methyl, group. Particularly preferred primary mine derivatives of formula I are those wherein each of R¹ and R² independently represents a hydrogen atom or a methyl group, y is 2 or 3, m is 0 and n is in the range 1 to 6.

The primary amine derivative may be a single compound of formula I or mixture of two or more compounds of formula I.

Examples of primary amine derivatives of formula I above include 3-dimethylamino-1-propylamine, diethylene triamine, triethylene tetramine, tetraethylene pentamine and pentaethylene hexamine which may be obtained from the Aldrich Chemical Company Ltd., (UK); hexaethylene heptamine which may be obtained from Union Carbide Corporation (USA); H₂N-C₂H₄-S-C₂H₄-NH-C₂H₄-NH₂ (see US Patent No. 3,362,996); and H₂N-(C₂H₄-O)ₚ-C₂H₄-NH₂ (p=1,2) and H₂N-(C₂H₄-S)_{q}-C₂H₄-NH₂ (q=1,2) which are listed in Beilsteins Handbuch der Organischen Chemie (Springer-Verlag, Hamburg, Viertes Erganzungswerke).

Particularly preferred are 3-dimethylamino-1-propylamine, diethylene triamine, tetraethylene pentamine, pentaethylene hexamine, hexaethylene heptamine, and mixtures of tetraethylene pentamine, pentaethylene hexamine and hexaethylene heptamine.

The primary amine derivatives of formula I above may be prepared by the methods of H.R. Ing and R.H.F. Manske, J. Chem. Soc., 1926, p. 2348 et seq.; D.S. Tarbell, N. Shakespeare, C.J. Claus and J.R. Bunnett, J. Am. Chem. Soc., 1946, 68, p. 1217 et seq.; R.C. O'Gee and H.M. Woodburn, J. Am. Chem. Soc., 1951, 73, p. 1370 et seq.; A.F. McKay, D.L. Garmaise and A. Halsaz, Can. J. Chem., 1956, 34, p. 1567 et seq.; Kirk-Othmer Encyclopedia of Chemical Technology, Volume 7 (Wiley Interscience, New York, 3rd edition, 1979) pp 580-602; US Patent No. 2,318,729; S. Gabriel, Berichte, 1891, 24, pp 1110-1121; F.P.J. Dwyer and F. Lions, J. Am. Chem. Soc., 1950, 72, pp 1545-1550; F.P.J. Dwyer, N.S. Gill, E.C. Gyarfas and F. Lions, J. Am. Chem. Soc., 1953, 75, pp 1526-1528; US Patent No. 3,362,996; or by processes analogous thereto.

The process according to the invention is conveniently carried out at a temperature in the range of from 80 to 250°C, preferably from 100 to 200°C, and especially from 110 to 180°C, advantageously 160 to 170°C.

The present process is conveniently carried out at a total pressure as determined at ambient temperature (20°C) of from 200 to 25,000 kPa preferably from 2,000 to 20,000 kPa, and especially from 4,000 to 8,000 kPa. It will be appreciated, however, that when the process is operated batchwise at a constant temperature, the pressure will fall during the course of reaction.

A source of cationic rhodium or, if present, ruthenium may be any material comprising rhodium or ruthenium which is capable of yielding cationic rhodium or ruthenium species. Examples of suitable sources include compounds of rhodium or ruthenium such as oxides; salts such as nitrates, sulphates, sulphonates (e.g. trifluoromethanesulphonates or p-toluenesulphonates), borates (e.g. tetrafluoroborates), phosphates, phosphonates (e.g. benzenephosphonates), carboxylates (e.g. acetates, propionates or butyrates), and acetylacetonates (e.g. Rh(CO)₂(acac) or Ru(acac)₃ where acac denotes an acetylacetonate anion); carbonyls (e.g. triruthenium dodecacarbonyl) and hydrides (e.g. HRh(CO)(Ph₃P)₃, H₂Ru₄(CO)₁₃, or H₄Ru₄(CO)₁₂).

The aromatic N-heterocyclic ligand used in the process of the present invention may be monodentate or multidentate containing a plurality of coordinative N-ring atoms. Preferably it is a bidentate ligand containing two coordinative nitrogen atoms. It will be appreciated that the ligand should be inert. The ligand may comprise one or more, e.g. up to 6, preferably up to 4, substituents on the N-heterocyclic ring or rings, which substituents may be linked together to form condensed ring systems such as phenanthroline or quinoline. Examples of substituents include carboxyl, alkyl, alkoxy, alkylthio, alkoxycarbonyl, alkylamino, dialkylamino, cycloalkyl and aryl groups. Any alkyl moiety in such substituents may be linear or branched and preferably contains up to 6, more preferably up to 4, carbon atoms. A cycloalkyl substituent group preferably contains from 3 to 8, more preferably 3 to 6, carbon atoms. An aryl substituent group may be any aromatic hydrocarbon group, especially a phenyl or napthyl group.

The expression "aromatic N-heterocyclic ligand" as used herein indicates an organic ring compound containing at least one imino N-ring atom and a plurality of double bonds in the ring structure, such that the electron density is at least partially delocalised. The ring may be five-, six- or seven-membered, and may contain, besides carbon and nitrogen atoms, other hetero atoms such as oxygen or sulphur. Two aromatic N-heterocyclic rings may be linked together to form a bidentate ligand such as in 2,2'-bipyridine or 1,10-phenanthroline.

Preferably the aromatic N-heterocyclic ligand is an optionally substituted pyridine, quinoline, 2,2'-bipyridine or 1,10-phenanthroline.

Examples of aromatic N-heterocyclic ligands are pyridines, e.g. pyridine, 3-methylpyridine, 4-methylpyridine, 2,6-dimethylpyridine, 4-ethylpyridine, 2-methoxypyridine, 2-dimethylaminomethylpyridine, 2-pyridinecarboxylic acid; quinolines, e.g. quinoline or 2-methylquinoline; 2,2'-bipyridines, e.g. 2,2'-bipyridine, 4,4'-di-t-butyl-2,2'-bipyridine, 4,4'-diphenyl-2,2'-bipyridine, 4,4'-dicarboxyl-2,2'-bipyridine, 3,3'-dimethyl-2,2'-bipyridine, 4,4'-dimethyl-2,2'-bipyridine, 5,5'-dimethyl-2,2'-bipyridine or 6,6'-dimethyl-2,2'-bipyridine; 1,10-phenanthrolines, e.g. 1,10-phenanthroline, 4,7-dimethyl-1,10-phenanthroline, 3,4,7,8-tetramethyl-1,10-phenanthroline, 4,7-diphenyl-1,10-phenanthroline, or 3,4,5,6,7,8-hexamethyl-1,10-phenanthroline. Further suitable ligands include 2-(2-pyridyl)benzimidazole, 3-(2-pyridyl)-5,6-diphenyl-1,2,4-triazine, the monosodium salt of 3-(2-pyridyl)-5,6-dipheny1-1,2,4-triazine-p,p'-disulphonic acid and the disodium salt of 4,4'-dicarboxyl-2,2'-bipyridine.

Particularly preferred ligands are 2,2'-bipyridine, 4,4'-diphenyl-2,2'-bipyridine, 4,4'-dimethyl-2,2'-bipyridine, 4,4'-dicarboxyl-2,2'- bipyridine, 1,10-phenanthroline, 3,4,7,8-tetramethyl- 1,10-phenanthroline and 4,7-diphenyl-1,10-phenanthroline.

The source of an anion other than a halide may be a salt or an acid. It may also be a metal complex, for example a rhodium or ruthenium complex. The anion is preferably a nitrate, sulphate, sulphonate, borate, phosphate, phosphonate, carboxylate or acetylacetonate anion. Preferably the source of anion is a complex or salt of rhodium or ruthenium, or an acid.

The number of moles of aromatic N-heterocyclic ligand per gram atom of rhodium is preferably in the range of from 0.5 to 1000, more preferably from 0.75 to 50, and especially from 1 to 10.

The number of moles of anion per gram atom of rhodium is preferably at least 0.5, more preferably in the range of from 1 to 100, and especially from 2 to 50.

The number of gram atoms of rhodium used per mole of selectively hydrogenated star-shaped polymer is not critical. It is preferably in the range of from 10⁻⁶ to 10² gram atoms rhodium per mole of selectively hydrogenated star-shaped polymer.

The ratio of the number of gram atoms of rhodium to ruthenium preferably ranges from 100:1 to 1:100, more preferably from 100:1 to 1:10, and especially from 10:1 to 1:1.

The CO to H₂ ratio preferably ranges from 1:10 to 10:1, more preferably from 1:5 to 5:1.

The process of the invention is carried out in the presence of a solvent. Typical examples of solvents are ethers such as diethyleneglycol dimethylether (diglyme), 1,4-dioxane or tetrahydrofuran; amides such as dimethylformamide or dimethylacetamide; alcohols such as hexanol or tetraethylene glycol; esters such as ethyl acetate; and hydrocarbons such as hexane, cyclohexane, toluene and the xylenes.

The present invention further provides secondary amines obtainable by the process of the invention.

The secondary amines according to the present invention may be used as additives in lubricating oils. Accordingly, the present invention further provides a lubricating oil composition comprising a major amount of a lubricating oil and a minor amount, preferably from 0.1 to 5%w, especially from 0.5 to 2%w, (based on the total composition) of a secondary amine according to the present invention.

Suitable lubricating oils are natural, mineral or synthetic lubricating oils.

Natural lubricating oils include animal and vegetable oils, such as castor oil. Mineral oils comprise the lubricating oil fractions derived from crude oils, coal or shale, which fractions may have been subjected to certain treatments such as clay-acid, solvent or hydrogenation treatments. Synthetic lubricating oils include synthetic polymers of hydrocarbons, modified alkylene oxide polymers, and ester lubricants, which are known in the art. These lubricating oils are preferably crankcase lubricating oils for spark-ignition and compression-ignition engines, but include also hydraulic lubricants, metal-working fluids and automatic transmission fluids.

The lubricating compositions according to the present invention may contain various other additives, known in the art, such as viscosity index improvers, e.g. linear or star-shaped polymers of a diene such as isoprene or butadiene, or a copolymer of such a diene with optionally substituted styrene. These copolymers are suitably block copolymers and are preferably hydrogenated to such an extent as to saturate most of the olefinic unsaturation. Other suitable additives include dispersant V.I. improvers such as those based on block copolymers, or polymethacrylates, extreme pressure/anti-wear additives such as zinc or sodium dithiophosphates, ashless dispersants such as polyolefin-substituted succinimides, e.g. those described in GB-A-2 231 873, anti-oxidants, friction modifiers or metal-containing detergents such as phenates, sulphonates, alkylsalicylates or naphthenates, all of which detergents may be overbased.

The secondary amines according to the present invention may also be used as additives in fuels. Accordingly, the present invention further provides a fuel composition comprising a major amount of a fuel and a minor mount, preferably from 0.1 to 5%w, especially from 0.5 to 2%w, (based on the total composition) of a secondary amine according to the present invention.

Suitable fuels include hydrocarbon base fuels boiling essentially in the gasoline boiling range from 30 to 230°C. These base fuels may comprise mixtures of saturated, olefinic and aromatic hydrocarbons. They can be derived from straight-run gasoline, synthetically produced aromatic hydrocarbon mixtures, thermally or catalytically cracked hydrocarbon feedstocks, hydrocracked petroleum fractions or catalytically reformed hydrocarbons.

The lubricating oil or fuel compositions according to the present invention may conveniently be prepared by blending an additives concentrate into the lubricating base oil or fuel. Such a concentrate generally comprises an inert carrier fluid and one or more additives in a concentrated form. In the preparation of the lubricating oil compositions according to the invention, the concentrate conveniently contains a lubricating oil as inert carrier fluid, whilst in the preparation of the fuel compositions according to the invention, the concentrate conveniently contains a fuel as inert carrier fluid. Hence the present invention further provides an additive concentrate comprising an inert carrier fluid and from 10 to 80%w, based on the total concentrate, of a secondary amine according to the present invention.

The invention will be further understood from the following illustrative example.

### Example

A solution of 50g "SHELLVIS 200" (Trade Mark) star-shaped polymer in cyclohexane (to 27% polymer content) ("SHELLVIS 200" star-shaped polymer is a star-shaped polymer comprising hydrogenated polyisoprene arms having Mₙ=35,000 coupled with divinylbenzene according to the procedure of GB-A-1,575,507) was introduced into a 250ml "Hastelloy C" (Trade Mark) metal autoclave together with 15mmol 3-dimethylamino-1-propylamine, 50ml tetrahydrofuran (THF) and, as catalyst, 0.1mmol Ru(acac)₃/0.2mmol Rh(CO)₂(acac)/1.2mmol 2,2'-bipyridine (acac denotes an acetylacetonate anion). The autoclave was then pressurised with a mixture of carbon monoxide and hydrogen (P_{CO}=3000 kPa, P_{hydrogen}=3000 kPa as determined at ambient temperature (20°C)) and the contents of the autoclave heated, with stirring, at 165°C for a period of 15 hours. After cooling, the product-containing THF-solution was added to ethanol and the resulting precipitate of product was filtered, washed with ethanol and subsequently dried at 80°C under nitrogen. The product was then dissolved in toluene, precipitated in ethanol, and was filtered, washed and dried as before. The product was then analysed for active matter and for basic-N content.

| Results | |
|---|---|
| Star polymer conversion (% mol/mol) | 44 |
| Basic-N (% N) | 0.10 |

The product is useful as a detergent/dispersant additive in lubricating oil and fuel compositions.

## Claims

1. A process for the preparation of a secondary amine which comprises reacting carbon monoxide, hydrogen, a selectively hydrogenated star-shaped polymer comprising a polyvinylaromatic nucleus and at least 4 polymeric arms linked to said nucleus wherein said polymeric arms are selected from the group consisting of:
(i) hydrogenated homopolymers and hydrogenated copolymers of conjugated dienes;
(ii) hydrogenated copolymers of conjugated dienes and monoalkenyl arenes; and
(iii) mixtures thereof;
and a primary amine derivative having a terminal -NH₂ group and a terminal -NR¹R² group, wherein each of R¹ and R² independently represents a hydrogen atom or an alkyl group; in the presence of a suitable solvent and a catalyst system comprising a source of cationic rhodium; optionally a source of cationic ruthenium; an aromatic N-heterocyclic ligand and a source of an anion other than a halide.

2. A process according to claim 1, wherein the primary amine derivative is a compound of the general formula: 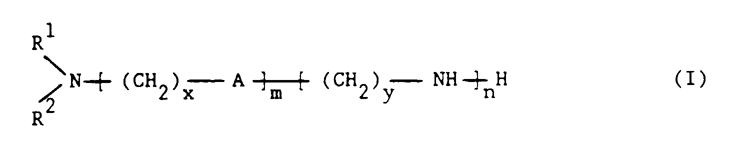 wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and R¹ and R² are as defined in claim 1.

3. A process according to claim 1 or claim 2 which is carried out at a total pressure in the range from 4,000 to 8,000 kPa.

4. A process according to any one of the preceding claims, wherein the source of cationic rhodium or ruthenium is an oxide, salt, hydride or carbonyl.

5. A process according to any one of the preceding claims, wherein the aromatic N-heterocyclic ligand is an optionally substituted pyridine, quinoline, 2,2'-bipyridine or 1,10-phenanthroline.

6. A process according to any one of the preceding claims, wherein the anion is a nitrate, sulphate, sulphonate, borate, phosphate, phosphonate, carboxylate or acetylacetonate anion.

7. A secondary amine obtainable by a process as claimed in any one of the preceding claims.

8. A lubricating oil composition comprising a major amount of a lubricating oil and a minor amount of a secondary amine as claimed in claim 7.

9. A fuel composition comprising a major amount of a fuel and a minor amount of a secondary amine as claimed in claim 7.

10. An additive concentrate comprising an inert carrier fluid and from 10 to 80%w, based on the total concentrate, of a secondary amine as claimed in claim 7.

## Patentansprüche

1. Verfahren zur Herstellung eines sekundären Amins, das ein Umsetzen von Kohlenmonoxid, Wasserstoff, einem selektiv hydrierten sternförmigen Polymer mit einem Gehalt an einem polyvinylaromatischen Kern und an wenigstens 4, an diesen Kern gebundenen polymeren Armen, worin die polymeren Arme aus der Gruppe ausgewählt sind, die aus:
(i) hydrierten Homopolymeren und hydrierten Copolymeren von konjugierten Dienen;
(ii) hydrierten Copolymeren von konjugierten Dienen und Monoalkenylarenen; und
(iii) Gemischen hievon besteht;
und einem primären Aminderivat mit einer endständigen -NH₂-Gruppe und einer endständigen -NR¹R²-Gruppe, worin jeder der Reste R¹ und R² unabhängig ein Wasserstoffatom oder eine Alkylgruppe darstellt; in Anwesenheit eines geeigneten Lösungsmittels und eines Katalysatorsystems umfaßt, das eine Quelle für kationisches Rhodium; gegebenenfalls eine Quelle für kationisches Ruthenium; einen aromatischen N-heterocyclischen Liganden und eine Quelle für ein von einem Halogenid unterschiedliches Anion umfaßt.

2. Verfahren nach Anspruch 1, worin das primäre Aminderivat eine Verbindung der allgemeinen Formel: 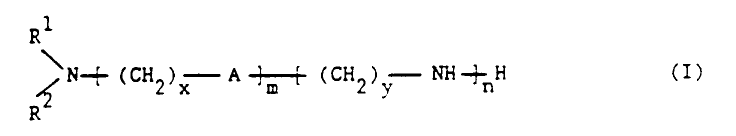 ist, worin jedes x im Bereich 1 bis 3 liegt, jedes y im Bereich 1 bis 3 liegt, jedes A unabhängig für -O- oder -S- steht, m den Wert 0, 1 oder 2 aufweist und n im Bereich 1 bis 10 liegt, und worin dann, wenn m für 1 steht, n den Wert 1 hat und A für -O- oder -S- steht, oder n den Wert 2 hat und A für -S- steht, und dann, wenn m 2 bedeutet, n den Wert 1 hat; und R¹ und R² wie in Anspruch 1 definiert sind.

3. Verfahren nach Anspruch 1 oder 2, das bei einem Gesamtdruck im Bereich von 4.000 bis 8.000 kPa ausgeführt wird.

4. Verfahren nach einem der vorstehenden Ansprüche, worin die Quelle für kationisches Rhodium oder Ruthenium ein Oxid, Salz, Hydrid oder Carbonyl ist.

5. Verfahren nach einem der vorstehenden Ansprüche, worin der aromatische N-heterocyclische Ligand ein gegebenenfalls substituiertes Pyridin, Chinolin, 2,2'-Bipyridin oder 1,10-Phenanthrolin ist.

6. Verfahren nach einem der vorstehenden Ansprüche, worin das Anion ein Nitrat-, Sulfat-, Sulfonat-, Borat-, Phosphat-, Phosphonat-, Carboxylat- oder Acetylacetonatanion ist.

7. Sekundäres Amin, erhältlich nach einem Verfahren, wie in einem der vorstehenden Ansprüche beansprucht.

8. Schmierölzusammensetzung mit einem Gehalt an einer Hauptkomponente eines Schmieröls und einer Nebenkomponente eines sekundären Amins, wie in Anspruch 7 beansprucht.

9. Treibstoffzusammensetzung mit einem Gehalt an einer Hauptkomponente eines Treibstoffes und einer Nebenkomponente eines sekundären Amins, wie in Anspruch 7 beansprucht.

10. Additivkonzentrat mit einem Gehalt an einem inerten Trägerfluid und an 10 bis 80 Gew.-%, bezogen auf das Gesamtkonzentrat, eines sekundären Amins, wie in Anspruch 7 beansprucht.

## Revendications

1. Procédé de préparation d'une amine secondaire, caractérisé en ce que l'on fait réagir le monoxyde de carbone, l'hydrogène et un polymère étoilé sélectivement hydrogéné, comprenant un noyau polyvinylaromatique et au moins quatre bras polymériques liés au noyau précité, où lesdits bras polymériques sont choisis dans le groupe formé par :
(i) les homopolymères hydrogénés et les copolymères hydrogénés de diènes conjugués,
(ii) les copolymères hydrogénés de diènes conjugués et de monoalcénylarènes et
(iii) les mélanges de ces produits et un dérivé d'amine primaire possédant un radical -NH₂ terminal et un radical -NR¹R² terminal, où chacun des symboles R¹ et R² représente indépendamment un atome d'hydrogène ou un radical alkyle, en présence d'un solvant approprié et d'un système de catalyseur comprenant une source de rhodium cationique, éventuellement une source de ruthénium cationique, un ligand aromatique N-hétérocyclique et une source d'un anion autre qu'un anion halogénure.

2. Procédé suivant la revendication 1, caractérisé en ce que le dérivé d'amine primaire est un composé de la formule générale : 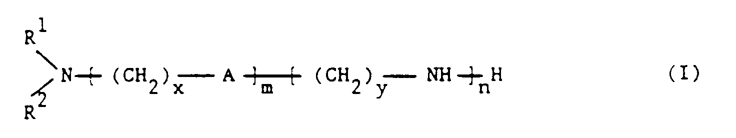 dans laquelle chaque symbole x varie de 1 à 3, chaque symbole y varie de 1 à 3, chaque symbole A représente indépendamment -O- ou -S-, m est égal à 0, 1 ou 2 et n a une valeur qui varie de 1 à 10 et, lorsque m est égal à 1, n est égal à 1 et A représente -O- ou -S-, ou bien n est égal à 2 et A représente -S- et, lorsque m est égal à 2, n est égal à 1 et R¹ et R² possèdent les mêmes significations que celles qui leur ont été attribuées dans la revendication 1.

3. Procédé suivant la revendication 1 ou la revendication 2, caractérisé en ce qu'on l'entreprend sous une pression totale qui varie de 4.000 à 8.000 kPa.

4. Procédé suivant l'une quelconque des revendications précédentes, caractérisé en ce que la source de rhodium ou de ruthénium cationique est un oxyde, un sel, un hydrure ou un carbonyle.

5. Procédé suivant l'une quelconque des revendications précédentes, caractérisé en ce que le ligand N-hétérocyclique aromatique est une 1,10-phénanthroline, une 2,2'-bipyridine, une quinoléine ou une pyridine, éventuellement substituée.

6. Procédé suivant l'une quelconque des revendications précédentes, caractérisé en ce que l'anion est un anion nitrate, sulfate, sulfonate, borate, phosphate, phosphonate, carboxylate ou acétylacétonate.

7. Amine secondaire que l'on peut obtenir par mise en oeuvre du procédé suivant l'une quelconque des revendications précédentes.

8. Composition d'huile lubrifiante, caractérisée en ce qu'elle comprend une proportion majeure d'une huile lubrifiante et une proportion mineure d'une amine secondaire suivant la revendication 7.

9. Composition de combustible ou de carburant comprenant une proportion majeure d'un combustible ou carburant et une proportion mineure d'une amine secondaire suivant la revendication 7.

10. Concentré d'additifs comprenant un liquide servant de véhicule inerte et de 10 à 80% en poids, sur base du concentré total, d'une amine secondaire suivant la revendication 7.
